# EUROPEAN PATENT APPLICATION

(11) **EP 3 384 859 A1**
(43) Date of publication of application: **10.10.2018**
(21) Application number: 18160513.0
(22) Date of filing: 07.03.2018
(51) Int. Cl.: A61B 17/3207

(54) **THROMBECTOMY CATHETER DEVICE**

(30) Priority: 04.04.2017 US 201715478725
(71) Applicant: Covidien LP, Mansfield, MA 02048 (US)
(72) Inventor: Malhi, Arnaz, Watertown, CA 95403 (US); Brightbill, Jerry, Newton Center, MA 02459 (US); Evans, Stephen, Westford, MA 01886 (US); Robinson, Tim, Sandown, NH 03873 (US); Hutton, Daniel, Brighton, MA 02135 (US)
(74) Representative: Gray, James

(57) **Abstract**

A catheter includes an outer tubular body, a spiral member, and a disruptor. The outer tubular body defines a longitudinal axis and extends to a distal annular cutting edge. The outer tubular body defines a lumen having distal and proximal ends. The spiral member is supported within the outer tubular body and includes proximal and distal ends. The distal end of the spiral member is positionable distally beyond the cutting edge of the outer tubular body. The disruptor is supported within the outer tubular body proximally of the cutting edge of the outer tubular body.

## Description

### TECHNICAL FIELD

The present disclosure relates to a system for removing occlusive materials from within blood vessels, and more particularly, to thrombectomy catheter devices.

### BACKGROUND

Devices can be used to remove occlusive material from a body lumen to maintain the patency of the body lumen. These devices may be of the mechanical, electrical or chemical type. Occlusive material ranges from chronic clots, sub-acute clots, and acute clots, depending on age and material of the clot.

One challenge associated with these devices is the ability to effectively remove different occlusive material while at the same time minimizing the likelihood of causing damage to the body lumen. Accordingly, it would be desirable to provide a device capable of efficiently removing a variety of different occlusive materials including, e.g., acute or chronic clots, from a body lumen while minimizing the potential risk of causing incidental damage to the surrounding healthy tissue.

### SUMMARY

Accordingly, in an embodiment of the present disclosure, a catheter for removing occlusive material disposed within a vessel includes an outer tubular body. The catheter comprises a spiral member, a disruptor, and a drive member. The outer tubular body defines a longitudinal axis and extends to a distal annular cutting edge configured to cut occlusive material within a vessel. The outer tubular body defines a lumen therethrough. The lumen has a distal end portion and a proximal end portion. In some embodiments, the outer tubular body includes a flexible portion.

The spiral member is supported within the outer tubular body and is configured to engage the occlusive material disposed within the vessel and draw the occlusive material into the distal end portion of the lumen of the outer tubular body. The spiral member includes a proximal end portion and a distal end portion. The distal end portion of the spiral member can be positioned distally beyond the cutting edge of the outer tubular body. The proximal end portion of the spiral member is supported about the disruptor. In embodiments, an energy source can be operatively coupled to the spiral member. In some embodiments, the spiral member is formed of an electrically conductive material.

The disruptor is supported within the outer tubular body and is configured to break up the occlusive material drawn into the lumen of the outer tubular body by, e.g., the spiral member, and facilitate removal of the occlusive material from the distal end portion of the lumen of the outer tubular body. The disruptor can be disposed within the outer tubular body proximally of the cutting edge of the outer tubular body. In embodiments, the disruptor defines at least one passage for permitting passage of occlusive material therethrough. In some embodiments, the disruptor includes at least one aperture to permit passage of occlusive material into the lumen of the outer tubular body. The at least one aperture can have a sharpened edge to facilitate the disruption of occlusive material. In embodiments, the disruptor has a conical configuration.

A drive member is operatively coupled to at least one of the spiral member and the disruptor to rotate at least one of the spiral member and the disruptor in relation to the outer tubular body. In embodiments, the drive member is adapted to rotate the spiral member and disruptor in relation to each other. For example, the spiral member and the disruptor can be adapted to rotate in opposite directions. In embodiments, the drive member includes an Archimedes-type screw for facilitating the proximal movement of the occlusive material through the lumen of the outer tubular body.

In embodiments, the drive member includes a first inner tubular body and a second inner tubular body that may be rotatable at different speeds relative to one another. The disruptor is secured to the first inner tubular body and the spiral member is secured to the second inner tubular body. In some embodiments, at least two of the first inner tubular body, the second inner tubular body, and the outer tubular body, are rotatable in opposing directions.

In embodiments, the disruptor has a body including at least one helical segment. The at least one helical segment includes at least one cutting blade that extends radially outwardly from the at least one helical segment. In some embodiments, the outer tubular body includes at least one internal cutting blade that is positioned in alignment with the at least one cutting blade of the at least one helical segment. The at least one helical segment is rotatable to engage the at least one cutting blade of the at least one helical segment with the at least one cutting blade of the outer tubular body, thereby shearing the occlusive material. In embodiments, the outer tubular body defines at least one notch within a wall of the outer tubular body. The at least one cutting blade of the outer tubular body is secured within the at least one notch.

In some embodiments, a vacuum source communicates with the lumen of the outer tubular body to facilitate aspiration of occlusive material from the lumen when the disruptor breaks up the occlusive material. In embodiments, a bearing bracket supports at least one of the spiral member and the disruptor within the outer tubular body. In some embodiments, at least one of the spiral member, the outer tubular body, and the drive member are coated with polytetrafluoroethylene ("PTFE").

According to one embodiment, a catheter includes an outer tubular body, an internal cutting mechanism, and a vacuum source. The outer tubular body defines a longitudinal axis and has a proximal end portion and a distal end portion. The internal cutting mechanism is disposed within the outer tubular body. The internal cutting mechanism includes a disruptor and a spiral member. The spiral member includes a proximal end portion and a distal end portion. The proximal end portion of the spiral member is longitudinally aligned with the disruptor along the longitudinal axis of the outer tubular body. The distal end portion of the spiral member extends distally of the disruptor and the distal end portion of the outer tubular body. A distal end portion of the disruptor is disposed proximally of the distal end portion of the outer tubular body. The spiral member is rotatable to engage occlusive material disposed within a vessel. The disruptor is configured to break up the occlusive material engaged with the spiral member and direct the occlusive material to an aspirating lumen defined within one of the outer tubular body and the internal cutting mechanism. The vacuum source is in fluid communication with the aspirating lumen to facilitate the aspiration of the occlusive material from the aspirating lumen. The distal end portion of the outer tubular body defines an annular cutting edge and the outer tubular body may be rotatable to rotate the cutting edge in relation to the occlusive material.

In one aspect, a method for removing an obstruction within a vessel includes advancing a catheter within a vessel adjacent an occlusive material within the vessel, securing the catheter relative to the occlusive material with a spiral member of the catheter, coring the occlusive material with a cutting edge of an outer tubular body of the catheter, breaking up the occlusive material with a disruptor disposed completely within the outer tubular body of the catheter, and displacing the occlusive material to a proximal end portion of the catheter.

Embodiments can include one or more of the following advantages.

The catheter can be guided to occlusive material within a vessel, for example, a chronic total occlusion, using standard endovascular techniques. The spiral member can be rotated to engage the occlusive material to draw the occlusive material to the distal annular cutting edge of the outer tubular member. The outer tubular body can be rotated in an opposite direction to the spiral member to assist in coring the occlusive material with the distal annular cutting edge. The rotation of the disruptor and drive member relative to the outer tubular member causes the occlusive material to be broken up by the cutting blades of the outer tubular member and the disruptor so that the broken up occlusive material can be advanced proximally and, in some cases, out of the vessel.

In some embodiments, an Archimedes-type screw, a vacuum source, and/or positive pressure provided by the rotation of the disruptor and/or outer tubular member can be provided to assist in removing occlusive material from the vessel.

Other aspects, features, and advantages will be apparent from the description, the drawings, and the claims that follow.

### BRIEF DESCRIPTION OF THE DRAWINGS

FIG. 1 is a schematic view of a thrombectomy catheter of the present disclosure illustrating a handle, an outer tubular body extending from the handle, and a vacuum source and a fluid source in communication with the outer tubular body.
FIGS. 2 and 3 are enlarged perspective views of a distal end portion of the outer tubular body of the thrombectomy catheter shown in FIG. 1, with an internal cutting mechanism removed for clarity.
FIG. 4 is a perspective view of the distal end portion of the thrombectomy catheter shown in FIG. 1, with the outer tubular body of the thrombectomy catheter depicted in cross-section.
FIG. 5 is a cross-sectional view of the distal end portion of the thrombectomy catheter shown in FIG. 4.
FIG. 6 is a perspective view of the distal end portion of the thrombectomy catheter of FIG. 1, with the entire outer tubular body of the catheter removed for clarity.
FIG. 7 is a perspective view of a distal end portion of another thrombectomy catheter of the present disclosure, with a distal end portion of an outer tubular body of the catheter shown in cross-section.
FIG. 8 is a perspective view of a proximal region of yet another thrombectomy catheter of the present disclosure, with a portion of an outer tubular body of the catheter depicted in phantom.
FIG. 9 is a side view illustrating still another thrombectomy catheter of the present disclosure, with portions of the catheter depicted in cross-section.

### DETAILED DESCRIPTION

As used herein, the term "clinician" refers to a doctor, nurse, or other care provider and may include support personnel. The terms "proximal" or "trailing" each refer to the portion of a structure closer to a clinician, and the terms "distal" or "leading" each refer to a portion of a structure farther from the clinician. As used herein, the term "subject" refers to a human patient or other animal. The term "lumen" refers to any lumen within the body, either natural or artificial, such as, for example, blood vessels, blood vessel grafts, fistulas, and the like. As used herein, the term "occlusion" refers to any partial or total blockage of a lumen, such as, for example; thrombus, atheromas, plaque, tumors, and the like. The term "disrupt" refers to cutting, emulsing, coring, crushing, shredding, separating, disintegrating, breaking-up, rupturing, and the like.

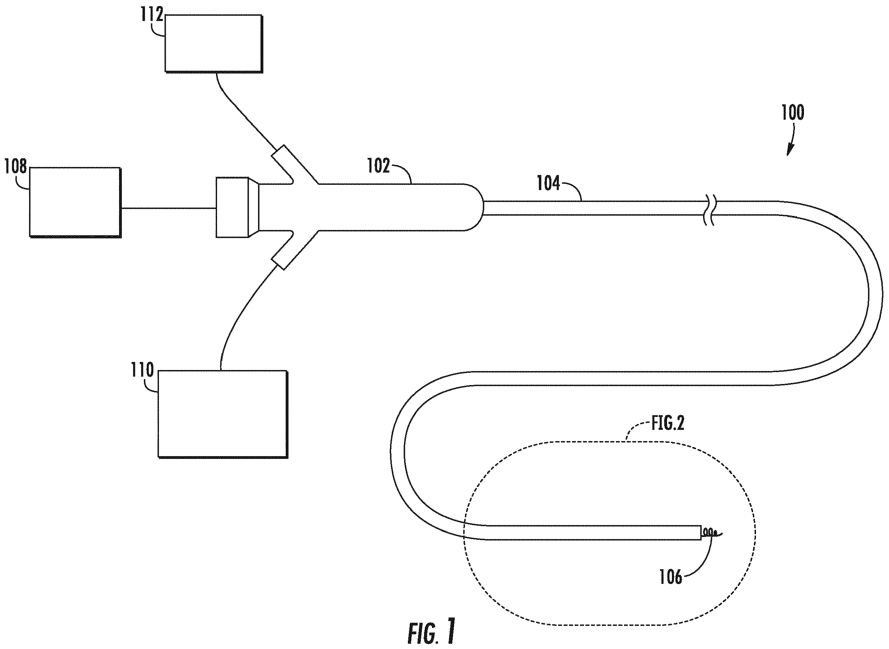

With reference to FIG. 1, a catheter 100 in accordance with the present disclosure is illustrated. The catheter 100 includes a handle 102, an outer tubular body 104 that extends distally from the handle 102, and an internal cutting mechanism 106, which is at least partially disposed within the tubular body 104.

The handle 102 may be dimensioned for engagement by the user. The handle 102 can incorporate, or be coupled to, a drive unit depicted schematically as reference numeral 108. The drive unit 108, which can include any suitable mechanical and/or electrical component, is coupled to, and activates, either, or both, the tubular body 104 and/or the internal cutting mechanism 106 to move as described below. In embodiments, the drive unit 108 includes an energy source that delivers energy (e.g., electrical, thermal, rotational, mechanical vibration, etc.) to the tubular body 104 and/or cutting mechanism 106. The handle 102 can include any number and/or types of knobs, switches, buttons, etc. that are manually and/or autonomously operable to impart any suitable mechanical and/or electrical rotational movement to any of the components of the catheter 100.

A vacuum source 110 and/or a fluid source 112 may each be coupled to the handle 102 and in fluid communication with the tubular body 104. The vacuum source 110 communicates negative pressure to the tubular body 104 to remove material, e.g., occlusive material, which may be disrupted by the tubular body 104 and/or the cutting mechanism 106. The fluid source 112 can deliver lytics or an irrigant such as saline, before, during, and/or after activation of the cutting mechanism 106.

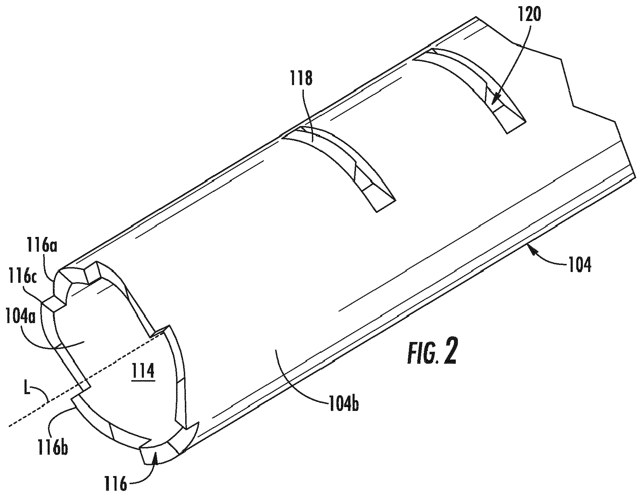

Referring now to FIG. 2, which depicts a distal end portion of the tubular body 104, the tubular body 104 includes an inner surface 104a and an outer surface 104b. The inner surface 104a of the tubular body 104 defines a lumen 114. The tubular body 104 defines a longitudinal axis "L" and extends to, or terminates at, a cutting edge 116 at the distal end face of the tubular body 104. The cutting edge 116 may be at least partially annular in configuration. In some embodiments, the cutting edge 116 is completely annular. The cutting edge 116 is dimensioned to disrupt occlusive material within a vessel upon rotation of the tubular body 104. The cutting edge 116 may include a stepped cutting surface 116a having one or more steps 116b defined therein, with each step 116b extending to a sharpened edge 116c. In other embodiments, the cutting edge 116 may include any suitable cutting arrangement that is dimensioned to disrupt occlusive material. For example, the cutting edge 116 can include any number of steps, ridges, edges, serrations or the like, and can have any suitable geometry and/or dimension. Alternatively, the cutting edge 116 may not include any steps or serrations, but instead may include a sharpened, annular distal edge.

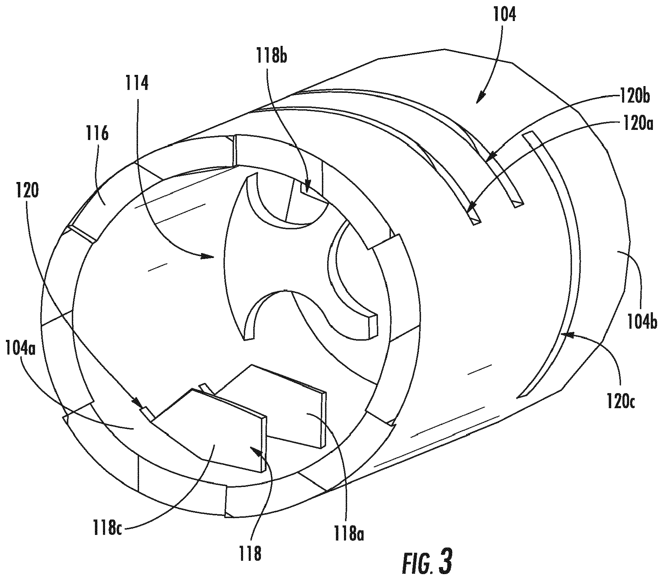

With reference to FIG. 3, the distal end portion of the tubular body 104 may further include a plurality of cutting blades 118 that extend from the inner surface 104a of the tubular body 104 into the lumen 114. Each cutting blade 118 may have any suitable geometry and/or dimension to disrupt occlusive material. Each cutting blade 118 maybe attached to the inner surface 104a of the tubular body 104 by securing each cutting blade 118 within a notch 120 defined in the tubular body 104 using known fastening techniques (e.g., welding, molding, crimping, adhesion, and/or the like). Each notch 120 can have any suitable geometry and/or dimension. Each of the cutting blades 118 and/or the notches 120 can be radially and/or longitudinally offset, and/or radially and/or longitudinally aligned, along the tubular body 104 relative to the other cutting blades 118 and/or the notches 120. For example, a notch 120a is radially aligned with, and longitudinally offset from, a notch 120b, while a notch 120c is radially and longitudinally offset from the notches 120a and 120b. Similarly, a cutting blade 118a is longitudinally aligned with, and radially offset from, a cutting blade 118b, while a cutting blade 118c is longitudinally offset from the cutting blades 118a and 118b, while radially aligned with the cutting blade 118a and radially offset from the cutting blade 118b. As an alternative, the cutting blades 118 may be monolithically formed with the tubular body 104.

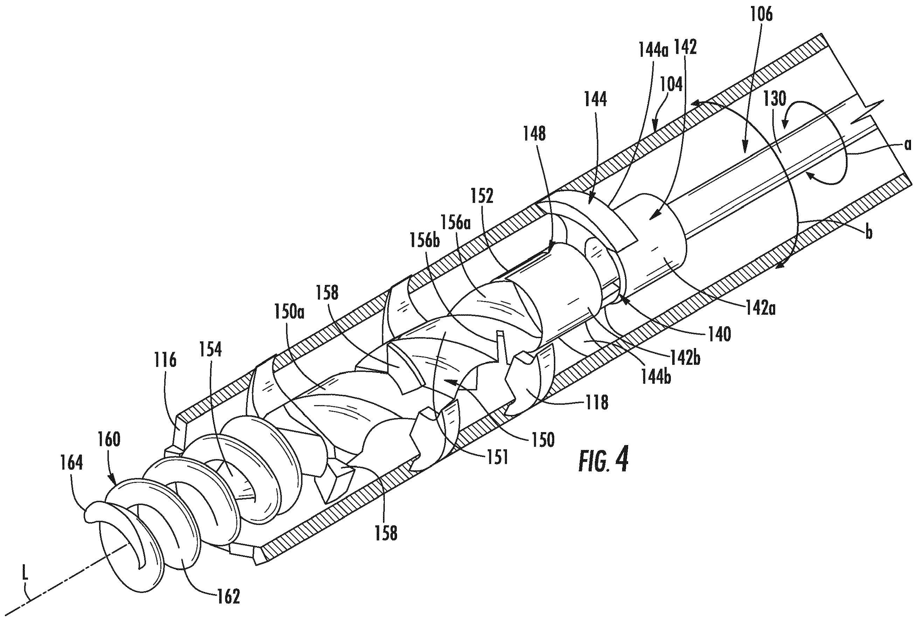

With reference to FIGS. 4-6, the internal cutting mechanism 106 within the tubular body 104 of the catheter 100 will be discussed. The internal cutting mechanism 106 may include, from proximal to distal, a drive member 130 (or a plurality of drive members), a bearing assembly 140, a disruptor 150 coupled to the drive member 130 by the bearing assembly 140, and a spiral member 160 which is coupled to the disruptor 150. The drive member 130 is operatively coupled to the drive unit 108 associated with the handle 102 of the catheter 100. The drive member 130 is adapted to rotate upon activation of the drive unit 108 to cause rotation of the disruptor 150 and/or the spiral member 160. The drive member 130 can be rigid and/or partially flexible (e.g., a hollow torque cable).

The bearing assembly 140 is supported between the proximal end portion of the disruptor 150 and a distal end portion of the drive member 130. The bearing assembly 140 includes a coupling device 142 and a bearing support 144 that supports the coupling device 142. The bearing assembly 140, or components thereof, can be secured together using known fastening techniques.

With continued reference to FIGS. 4-6, the coupling device 142 includes a proximal segment 142a and a distal segment 142b that are joined by an intermediate segment 142c. Each of these segments can be integrally formed with, and/or separately connectable to, one or more of the other segments of the coupling device 142 using known fastening techniques. The coupling device 142 is configured to operatively couple the drive member 130 and the disruptor 150. For example, the coupling device 142 may define a first recess 146a in proximal segment 142a and a second recess 146b in distal segment 142b. The first recess 146a is dimensioned to receive a distal end portion of the drive member 130 and the second recess 146b is dimensioned to receive a proximal end portion of the disruptor 150. The coupling device 142 may also define a slot 148 dimensioned to receive the proximal end portion of disruptor 150.

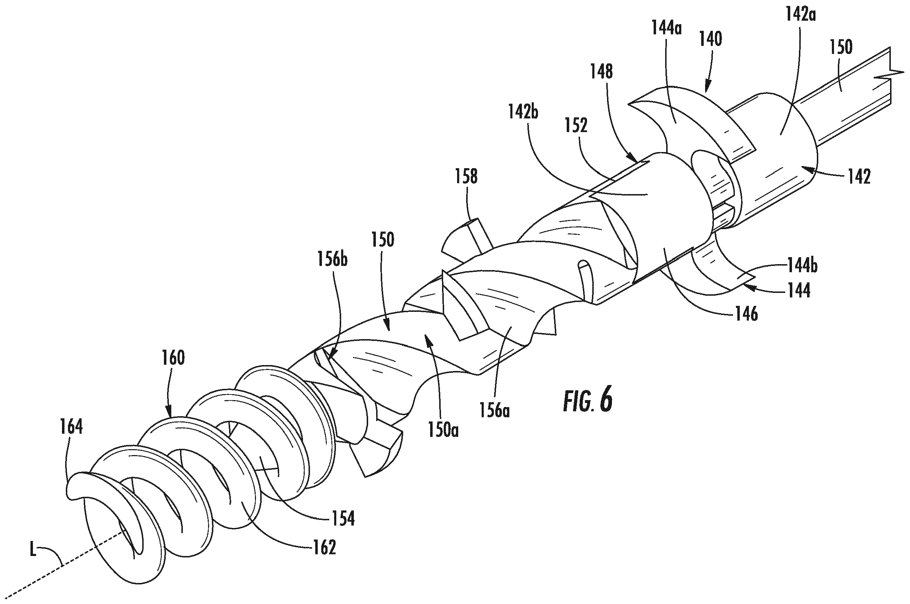

The bearing support 144 includes a first bearing bracket 144a and a second bearing bracket 144b. Although only two bearing brackets are illustrated in FIGS. 4 and 6, any suitable number of bearing brackets can be utilized. Each of the first and second bearing brackets 144a, 144b are positioned between the inner surface 104a of the tubular body 104 and the intermediate segment 142c of the coupling device 142 to support the coupling device 142 within the tubular body 104 so that the internal cutting mechanism 106 is maintained in coaxial alignment with longitudinal axis "L." The bearing brackets 144a, 144b can be secured to the inner surface 104a using known fastening techniques. In embodiments, the bearing brackets 144a, 144b can be secured within one or more of the notches 120 defined in the tubular body 104, such as the notch 120c (see FIG. 3) so that the bearing brackets 144a, 144b extend into the lumen 114 defined by the tubular body 104.

Referring still to FIGS. 4-6, the disruptor 150 includes a disruptor body 150a. The disruptor body 150a may have a key 152 that secures the disruptor 150 to the coupling device 142 of the bearing assembly 140. For example, the key 152 may be received within the slot 148 defined within the coupling device 142. The key 152 may have any suitable configuration and/or dimension including a planar arrangement. In embodiments, the key 152 includes surface texturing. The disruptor 150 is supported for rotational movement within the tubular body 104 through its connection to the coupling device 142 and the first and second bearing brackets 144a, 144b. While one bearing assembly 140 is shown, more than one bearing assembly 140 may be present. In such an embodiment, the other bearing assembly or assemblies 140 may be configured to only support the disruptor 150 within the lumen 114 defined by the tubular body 104 and not provide any coupling functions as described above.

The disruptor body 150a is configured to move and break up any occlusion brought into the lumen 114 defined by the tubular body 104. For example, in the disclosed embodiment, the disruptor body 150a defines an outer thread or screw configuration including one or more helical segments 151 extending distally from the key 152 to a distal tip 154. The outer thread or screw defines a spiral recess 156a along an outer surface of the disruptor body 150a, e.g., between the helical segments 151. The disruptor body 150a also defines a plurality of slots 156b at axially spaced locations along the disruptor body 150a that are dimensioned to facilitate breaking up any occlusive material. In some embodiments, each of the plurality of slots 156b is positioned in alignment with one or more of the cutting blades 118 of the tubular body 104. In various embodiments, the distal tip 154 of the disruptor 150 can be disposed proximally of the cutting edge 116 of the tubular body 104 so that the disruptor 150 is positioned completely within the tubular body 104. In other embodiments, the distal tip 154 of the disruptor 150 can be disposed distally of the cutting edge 116 of the tubular body 104 so that the disruptor 150 extends distally beyond the tubular body 104.

A plurality of cutting blades 158 may be supported at locations along the outer surface of the disruptor body 150a. These cutting blades 158 further help breakup or macerate any occlusive material. Each cutting blade 158 can have any suitable shape and/or dimension. Each cutting blade 158 can be integrally formed with the disruptor body 150a and/or can be separately connectable to the disruptor body 150 using known fastening techniques. Two or more of the plurality of cutting blades 158 can be longitudinally and/or radially offset (e.g., out of phase) relative to each other. Alternatively, and or additionally, two or more of the plurality of cutting blades 158 may be longitudinally and/or radially aligned (e.g., in phase) relative to each other. Each cutting blade 158 projects radially outwardly from the outer surface of the disruptor body 150a relative to the longitudinal axis "L." In embodiments, each cutting blade 158 is arranged in substantial longitudinal alignment, but slightly offset, with one of the blades 118 of the tubular body 104 to create a shearing action on the occlusive material when the respective cutting blades 158, 118 are moved into engagement.

With continued reference to FIGS. 4-6, a spiral member 160 may be secured to the disruptor 150. The spiral member 160 rotates with the disruptor 150 within the lumen 114 of outer tubular member 104. In embodiments, the distal tip 154 of the disruptor 150 is secured to a proximal end portion of the spiral member 160 using known fastening techniques. In embodiments, the proximal end portion of the spiral member 160 is integrally formed with the distal end portion of the disruptor 150. In some embodiments, the disruptor 150 and the spiral member 160 can be dimensioned to rotate with the outer tubular member 104 and/or independent of the outer tubular member 104, for example, at different speeds and/or directions.

The spiral member 160 has a substantially rigid spiral or helical body 162 that extends to a distal tip 164 in a corkscrew configuration such that adjacent winding segments of the helical body 162 are disposed in a spaced apart configuration. The distal tip 164 may be sharpened. A distal end portion of the spiral member 160 extends distally beyond the cutting edge 116 of the tubular body 104 and is longitudinally fixed relative to the annular cutting edge 116 of the outer tubular member 104.

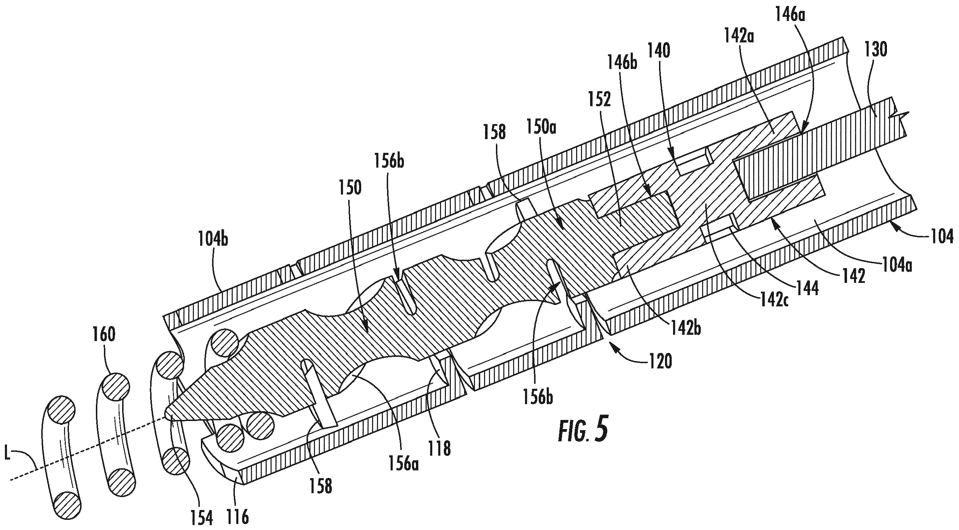

Although the helical body 162 is shown in FIG. 5 with a substantially circular cross-section that spirals about the longitudinal axis "L," the helical body 162 can have any suitable dimension and/or geometric cross-sectional configuration including oval, polygonal, and the like.

In embodiments, the spiral member 160 may be formed of an electrically conductive material and can be operatively coupled to an energy source associated, e.g., with the drive unit 108, such as an electrosurgical generator that conducts electrical energy through the spiral member 160 to facilitate electrical disruption of occlusive material.

In use, the catheter 100 is advanced to a position adjacent to occlusive material within a vessel and secured relative to the occlusive material by axially and/or rotationally moving the catheter 100 in relation to the occlusive material to embed the spiral member 160 into the occlusive material. The drive unit 108 can be actuated to impart rotational movement to the internal cutting mechanism 106 in either rotational direction (e.g., clockwise or counterclockwise), as depicted as arrow "a" (Fig. 4), so that while the spiral member 160 of the internal cutting mechanism 106 is engaged with the occlusive material within the vessel, axial and/or rotational movement of the catheter 100 and/or components thereof (e.g., the spiral member 160) brings the occlusive material and the cutting edge 116 of the tubular body 104 together to cause coring of the occlusive material. The spiral member 160 may pull the occlusive material into the lumen 114 and/or pull the distal end of the tubular body 104 towards and into the occlusive material. Additionally, or alternatively, the drive unit 108 can rotate the tubular body 104 in either rotational direction as depicted as arrow "b" (Fig. 4). The occlusive material can then be drawn into the distal end portion of the tubular body 104 through the lumen 114 so that the disruptor 150 and the cutting blades 118 that extend from the tubular body 104 cooperate to slice, cut, or disrupt the occlusive material through rotation of the disruptor 150 and the blades 158, and/or the tubular body 104. Upon disrupting the occlusive material, the disrupted occlusive material is then displaced (e.g., by virtue of the rotational movement of the tubular body 104 and/or the disruptor 150 of the internal cutting mechanism 106) to a proximal end portion of the catheter 100 to remove the occlusive material from the catheter 100, for example, with the vacuum source 110 when the vacuum source 110 is coupled to the catheter 100. Alternatively or additionally, the vacuum source 110 may facilitate movement of the occlusive material toward the proximal end portion of the catheter 100 when initially engaged by the spiral member 160 and/or the disruptor 150.

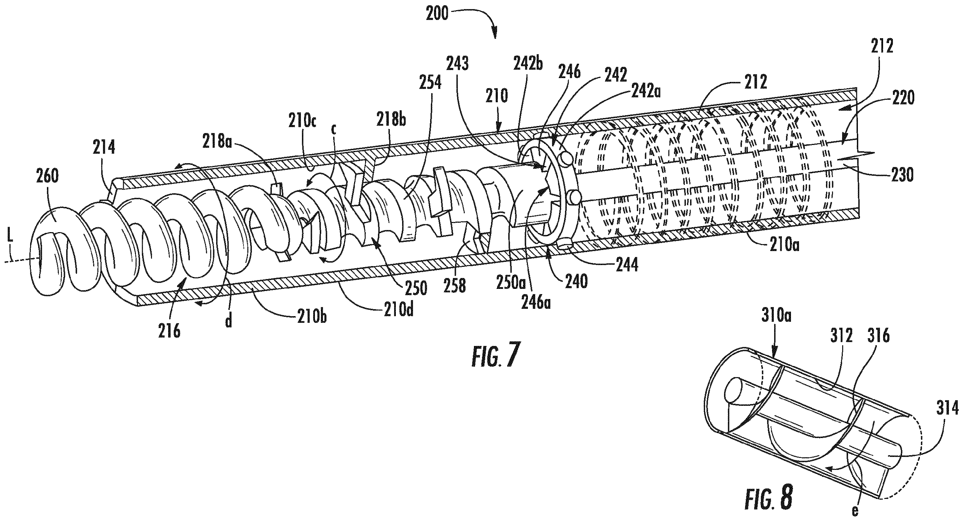

Turning now to FIG. 7, a distal end portion of a thrombectomy catheter of another embodiment of the present disclosure is shown and is generally referred to as a catheter 200. The catheter 200 includes an outer tubular body 210 that supports an internal cutting mechanism 220. The internal cutting mechanism 220 includes, from proximal to distal, a drive member 230, a bearing assembly 240, a disruptor 250, and a spiral member 260.

As illustrated in FIG. 7, the distal end portion of the outer tubular body 210 includes a proximal region 210a and a distal region 210b, which together define the outer tubular body 210. The proximal region 210a may include a flexible coil or coil members 212 embedded within the proximal region 210a. The distal region 210b extends to a distal annular cutting edge 214 and may have a different rigidity (e.g., more rigid) relative to the proximal region 210a to facilitate insertion of the catheter 200 into a body lumen.

The outer tubular body 210 extends to an annular cutting edge 214 and includes an inner surface 210c and an outer surface 210d. The inner surface 210c defines a lumen 216 which extends between the proximal and distal end portions of the outer tubular body 210. The outer tubular body 210 defines a plurality of notches 218a and includes a plurality of cutting blades 218b that are secured within the notches 218a using known fastening techniques. Alternately, the cutting blades 218b can be integrally formed with the outer tubular body 210.

The drive member 230, which is operatively coupled to a drive unit at a proximal end portion (as described above), is secured directly to a proximal end portion of the disruptor 250 at a distal end portion of the drive member 230. In various embodiments, the drive member 230 and disruptor 250 are an integral assembly, while in other embodiments, the drive member 230 and disruptor 250 are separate components secured to one another. More particularly, as seen in FIG. 7, the bearing assembly 240 is supported between a proximal end portion of the disruptor 250 and a distal end portion of the drive member 230 and includes an annular member 242 having an outer surface 242a and an inner surface 242b. A plurality of protuberances 244 are supported on the outer surface 242a of the annular member 242 in spaced apart relationship with adjacent protuberances 244. The protuberances 244 are positioned to fixedly engage the inner surface 210c of the tubular body 210 to secure the annular member 242 within the tubular body 210. A plurality of arms 246 extend radially inward from the inner surface 242b of the annular member 242 toward the longitudinal axis "L" in spaced apart relationship from adjacent arms 246 to define passages 243. Together, all of the arms 246 define a centrally disposed channel 246a that receives and engages the disruptor 250 to rotatably support the disruptor 250 within the tubular body 210.

The disruptor 250 includes a proximal end portion and a distal end portion. The proximal end portion of the disruptor 250 includes a cylindrical portion 250a that is secured to the distal end portion of the drive member 230 such that the disruptor 250 extends distally from the drive member 230 through the channel 246a defined by the bearing assembly 240. The disruptor 250 includes a disruptor body 254 with a spiral configuration. The distal end portion of disruptor 250 is secured to a proximal end portion of the spiral member 260 using known fastening techniques. The disruptor body 254 includes a plurality of cutting blades 258 that project radially outwardly from the outer surface of disruptor body 254 away from the longitudinal axis "L."

Each cutting blade 258 is arranged in substantial longitudinal alignment with one of the blades 218b of the outer tubular member 210 to create a shearing action when the respective blades 218b, 258 are moved into engagement. In particular, each cutting blade 258 engages with one of the blades 218b of the outer tubular member 210 to shear occlusive material drawn between the disruptor body 254 and the outer tubular body 210 to facilitate disruption and proximal displacement of occlusive material. Each cutting blade 258 can have any suitable geometry and/or dimension to disrupt occlusive material.

In use, similar to the catheter 100, the catheter 200 is advanced to a position adjacent to occlusive material within a vessel and secured relative to the occlusive material. The internal cutting mechanism 220 and/or the outer tubular body 210 are rotated to core the occlusive material and pull the cored occlusive material into the outer tubular body 210. The rotation of the internal cutting mechanism 220, in either rotational direction as depicted by arrow "c," and/or the rotation of the outer tubular body 210, in either rotational direction as depicted by arrow "d," breaks up the occlusive material and displaces the disrupted occlusive material to a proximal end portion of the catheter 200. The occlusive material can then be removed from the catheter 200.

Referring to FIG. 8, in another embodiment of the proximal region of the outer tubular body 210, referred to as a proximal region 310a, an Archimedes-type screw 316 is provided that spirals around a drive member 314. The Archimedes-type screw 316 is dimensioned to cooperate with an inner surface 312 of the proximal region 310a to facilitate proximal displacement of disrupted occlusive material through the catheter upon rotational movement of the drive member 314, as depicted by arrow "e," after the occlusive material is disrupted by the disruptor 250 and passed through the passages 243 defined by the bearing assembly 240. In embodiments, the Archimedes-type screw 316 can be spiraled around the drive member 314 in an opposite rotational direction of that depicted in FIG. 8 so that rotational movement of the drive member 314 in a direction opposite to that depicted by arrow "e" facilitates the proximal displacement of disrupted occlusive material. As can be appreciated, any of the presently disclosed catheters 100, 200 can be modified to include an Archimedes-type screw to enable proximal displacement and removal of disrupted occlusive material from the catheter.

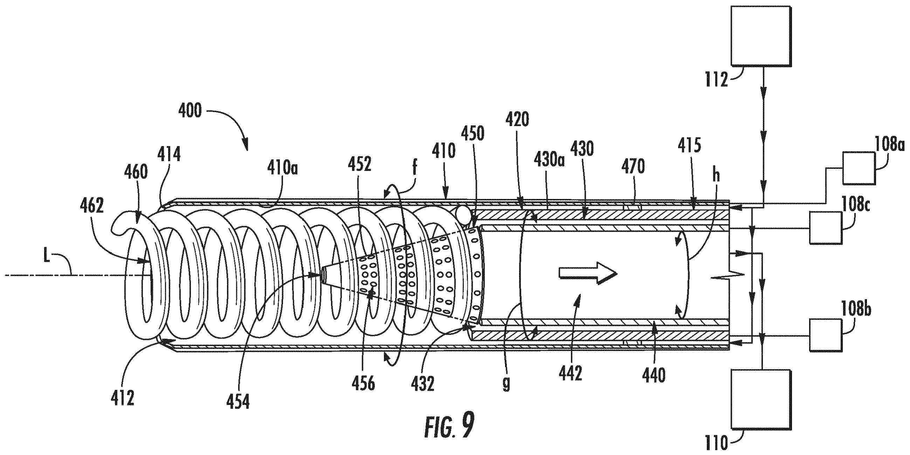

Referring now to FIG. 9, another embodiment of a thrombectomy catheter is illustrated which is generally referred to as a catheter 400. The catheter 400, which is operatively coupled to a fluid source and/or a vacuum source, such as vacuum and fluid sources 110, 112, includes an outer tubular body 410 and an internal cutting mechanism 420 supported within the outer tubular body 410. The internal cutting mechanism 420 includes, from proximal to distal, a first inner tubular body 430, a second inner tubular body 440, a disruptor 450, and a spiral member 460.

The outer tubular body 410 includes proximal and distal end portions and defines a lumen 412 that extends between the proximal and distal end portions. The outer tubular body 410 defines a longitudinal axis "L" and extends to a distal annular cutting edge 414 which is dimensioned to disrupt occlusive material within a vessel. The outer tubular body 410 can be coupled to a drive unit 108a that imparts rotational movement to the outer tubular body 410 in either rotational direction as depicted by arrow "f."

The first inner tubular body 430 is disposed within the lumen 412 of the outer tubular member 410 and has distal and proximal end portions. The distal end portion of the first inner tubular body 430 is secured to a proximal end portion of the spiral member 460 using known fastening techniques. In embodiments, the first inner tubular body 430 can be coupled to a drive unit 108b that imparts rotational movement to the first inner tubular body 430 in either rotational direction as depicted by arrow "g."

An outer surface 430a of the first inner tubular body 430 can be separated from an inner surface 410a of the outer tubular body 410 by any number of miniature stand-offs 470 to define an annular space 415 between the outer tubular body 410 and the first inner tubular body 430. Any number of the stand-offs 470 may be disposed in spaced apart relationship along the inner surface 410a of the outer tubular body 410. As can be appreciated, the outer surface 430a of the first inner tubular body 430 and/or the stand-offs 470 can have a lubricious surface to facilitate rotational movement of the first inner tubular body 430. The annular space 415 can be disposed in fluid communication with the fluid source 112. The fluid source 112 can infuse a fluid, for instance, saline, water, and/or a suitable lytic material, into the lumen 412 to provide positive flow for facilitating removal of occlusive material and replenishing the fluid removed from a body lumen through aspiration via the vacuum source 110. The inner surface of the first inner tubular body 430 defines a lumen 432 between the proximal and distal end portion of the first inner tubular body 430.

The second inner tubular body 440 is supported in the lumen 432 of the first inner tubular body 430 and includes distal and proximal end portions. The distal end portion of the second inner tubular body 440 is coupled to a proximal end portion of the disruptor 450 using known fastening techniques. The inner surface of the second inner tubular body 440 defines an aspirating lumen 442 that is in fluid communication with the vacuum source 110 to facilitate the aspiration of the occlusive material from the aspirating lumen 442. In embodiments, the second inner tubular body 440 can be coupled to a drive unit 108c that imparts rotational movement to the second inner tubular body 440 and the disruptor 450 in either rotational direction as depicted by arrow "h."

The first tubular body 430, the second tubular body 440, and/or the outer tubular body 410 can be rotated at different and/or the same speeds and/or different and/or the same directions relative to one another. In some embodiments, the separate driving units (e.g., the drive units 108a, 108b, 108c) coupled to the respective tubular bodies 410, 430, and/or 440 cause the bodies 410, 430, and/or 440 to rotate independently, and relative to one another, and in different rotational directions. Alternatively and/or additionally, the bodies 410, 430, and/or 440 can be operably coupled to any suitable gear assembly that causes the bodies 410, 430, and/or 440 to rotate relative to one another and/or at different speeds.

The disruptor 450 may include a substantially conically shaped body 452. The disruptor 450 extends into a channel 462 defined centrally through the spiral member 460. The conically shaped body 452 defines a substantially conical central passage 454 between distal and proximal end portions of the conically shaped body 452. The conical central passage 454 is disposed in fluid communication with the lumen 442 of the second inner tubular body 440 and is dimensioned to permit passage of occlusive material therethrough. A plurality of apertures 456 is defined within the outer surface of the conically shaped body 452 to permit passage of occlusive material into the aspiration lumen 442 of the second inner tubular body 440. Each aperture 456 can have a sharpened edge (e.g., like a cheese grater) to facilitate the disruption of occlusive material. Alternatively, in other embodiments, the disruptor 450 may include features similar to those described above in relation to the other catheters 100, 200 embodiments.

In use, similar to the catheters 100 and 200, the catheter 400 is advanced to a position adjacent to occlusive material within a vessel and secured relative to the occlusive material. The internal cutting mechanism 420 and/or the outer tubular body 410 are rotated to core the occlusive material and filter the cored occlusive material into the outer tubular body 410. The rotation of the internal cutting mechanism 420, including the disruptor 450 and the spiral member 460, and/or outer tubular body 410 disrupts the occlusive material and displaces the disrupted occlusive material toward a proximal end portion of the catheter 400. Shortly before, simultaneously therewith, or shortly thereafter, a vacuum source is coupled to catheter 400 at a proximal end portion thereof to draw the occlusive material in the proximal direction through the lumen 442 and/or apertures 456 to facilitate disruption and/or removal of the occlusive material from the catheter 400.

In some embodiments, the disruptor is not required. For example, the disruptor may not be necessary when used to remove acute clots. In this regard, a proximal end portion of the spiral member 460 can be directly secured to a distal end portion of the drive member or the second inner tubular body 440 using known fastening techniques.

Any surface of any of the components of these catheters can be coated with any suitable material, for example, PTFE.

Persons skilled in the art will understand that the structures and methods specifically described herein and illustrated in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be effected by one skilled in the art without departing from the scope or spirit of the disclosure. Additionally, it is envisioned that the elements and features illustrated or described in connection with one exemplary embodiment may be combined with the elements and features of another without departing from the scope of the present disclosure, and that such modifications and variations are also intended to be included within the scope of the present disclosure. Accordingly, the subject matter of the present disclosure is not to be limited by what has been particularly shown and described.

## Claims

1. A catheter for removing occlusive material disposed within a vessel, the catheter comprising:
an outer tubular body defining a longitudinal axis and extending to a distal annular cutting edge configured to cut occlusive material within a vessel, the outer tubular body defining a lumen therethrough, the lumen having a distal end portion and a proximal end portion;
a spiral member supported within the outer tubular body, the spiral member being configured to engage the occlusive material disposed within the vessel and draw the occlusive material into the distal end portion of the lumen of the outer tubular body, the spiral member including a proximal end portion and a distal end portion, the distal end portion of the spiral member being positionable distally beyond the cutting edge of the outer tubular body; and
a disruptor supported within the lumen of the outer tubular body, the disruptor being configured to break up the occlusive material drawn into the lumen of the outer tubular body and facilitate removal of the occlusive material from the distal end portion of the lumen of the outer tubular body, the disruptor being disposed within the outer tubular body proximally of the cutting edge of the outer tubular body.

2. The catheter of claim 1, wherein a vacuum source communicates with the lumen of the outer tubular body to facilitate aspiration of occlusive material from the lumen when the disruptor breaks up the occlusive material.

3. The catheter of claim 1, wherein the proximal end portion of the spiral member is supported about the disruptor.

4. The catheter of claim 1, wherein the disruptor has a body including at least one helical segment, the at least one helical segment including at least one cutting blade extending radially outwardly from the at least one helical segment.

5. The catheter of claim 4, wherein the outer tubular body includes at least one cutting blade that is positioned in general alignment with the at least one cutting blade of the at least one helical segment, the at least one helical segment being rotatable to engage the at least one cutting blade of the at least one helical segment with the at least one cutting blade of the outer tubular body to shear the occlusive material.

6. The catheter of claim 5, wherein the outer tubular body defines at least one notch within a wall of the outer tubular body, the at least one cutting blade of the outer tubular body being secured within the at least one notch.

7. The catheter of claim 1, wherein a bearing bracket supports at least one of the spiral member and the disruptor within the outer tubular body..

8. The catheter of claim 1, wherein the disruptor defines at least one passage for permitting passage of occlusive material therethrough.

9. The catheter of claim 1, wherein a drive member is operatively coupled to at least one of the spiral member and the disruptor to rotate at least one of the spiral member and the disruptor in relation to the outer tubular body.

10. The catheter of claim 9, wherein the drive member is adapted to rotate the spiral member and disruptor in relation to each other.

11. The catheter of claim 10, wherein the spiral member and the disruptor are configured to rotate in opposite directions.

12. The catheter of claim 9, wherein the drive member includes an Archimedes-type screw for facilitating the proximal movement of the occlusive material through the lumen of the outer tubular body.

13. The catheter of claim 9, wherein at least one of the spiral member, the outer tubular body, and the drive member is coated with PTFE.

14. The catheter of claim 11, wherein the drive member includes a first inner tubular body and a second inner tubular body that are rotatable at different speeds relative to one another, the disruptor being secured to the first inner tubular body and the spiral member being secured to the second inner tubular body.

15. The catheter of claim 14, wherein at least two of the first inner tubular body, the second inner tubular body, and the outer tubular body are rotatable in opposing directions.

16. The catheter of claim 1, wherein the disruptor has a conical configuration.

17. The catheter of claim 16, wherein the disruptor includes at least one aperture to permit passage of occlusive material into the lumen of the outer tubular body.

18. The catheter of claim 17, wherein the at least one aperture has a sharpened edge to facilitate the disruption of occlusive material.

19. The catheter of claim 1, further comprising an energy source operatively coupled to the spiral member, the spiral member being formed of an electrically conductive material.
